# EUROPEAN PATENT SPECIFICATION

(11) **EP 1 482 001 B1**
(45) Date of publication and mention of the grant of the patent: **27.04.2011**
(21) Application number: 04011762.4
(22) Date of filing: 18.05.2004
(51) Int. Cl.: C08G 63/82, C09D 175/16, C08G 63/91, C08G 63/87

(54) **Enzyme catalyzed polyesters and polyol polymers**
Enzym-katalysierte Polyester- und Polyolpolymere
Polymères de polyol et de polyester catalysés par enzyme

(30) Priority: 30.05.2003 US 448794
(43) Date of publication of application: 01.12.2004
(60) Divisional application: 10179153.1
(73) Proprietor: Armstrong World Industries, Inc., Lancaster Pennsylvania 17603 (US)
(72) Inventor: Tian, Dong, Lancaster, PA 17601 (US); Ross, Jeffrey S., Lancaster, PA 17603 (US); Price, Courtney R., Lancaster, PA 17603 (US)
(74) Representative: von Füner, Nicolai

(56) References cited:
- EP-A- 0 610 718
- EP-A- 1 048 683
- WO-A-99/46397
- US-A1- 2004 019 178

## Description

This invention is generally in the area of the preparation of polyester polyols by enzyme catalyst ring opening of lactones, as well as enzyme catalyzation of acrylated polyester polyols, and coating compositions including polyester polyols
and/or acrylated polyester polyols prepared by enzyme catalyst, providing stable coatings that offer several advantages relative to the use of heavy metal or strong acid catalysts to prepare the polyester polyols.

A limitation of using acidic and/or heavy metal catalysts is that the residues of these catalysts can accelerate the decomposition of the resulting polymers. It would be advantageous to provide methods for generating polyester polyols, including acrylated polyester polyols, using enzymatic catalysts, and providing coating compositions including these acrylated polyester polyols or components derived from these acrylated polyester polyols that are essentially free from strong acid or heavy metal residues. The present invention provides such methods and compositions.

In EP 0 610 718 A, a method for ring-opening polymerization in the presence of an alcohol and a lipase catalyst is disclosed. EP 0 610 718 A does not teach the use of an unsaturated polymerization initiator.

A method for producing linear polyesters made of carboxylic acids and polyhydric alcohols in the presence of a lipase is disclosed in WO 99/46397 A. However, this publication does not teach the incorporation of a polymerisation initiator with ethylenic unsaturation into the polymer.

The subject matter of the present invention is a polyester polyol composition comprising a polyester polyol comprising a plurality of moieties derived from a compound selected from the group consisting of a lactone, a lactide, a glycolide, and combinations thereof, wherein at least one of the plurality of moieties is covalently linked via an ester linkage to the hydroxy moiety of a hydroxy moiety-containing polymerization initiator, the hydroxy moiety-containing polymerization initiator comprising ethylenic unsaturation, and an enzyme catalyst.

Methods for synthesizing saturated and unsaturated polyester polyols are disclosed. The methods are environmentally-friendly and provide environmentally-friendly compositions that do not contain heavy metals or strong acid catalyst residues such as are present when heavy metals and/or strong acids are used as catalysts to manufacture these materials.

In one embodiment, the method involves the ring-opening polymerization of lactones, lactides and/or glycolides using hydroxy moiety-containing polymerization initiators, which initiators can include one or more double bond-containing moieties such as (meth)acrylates. As used herein, "(meth)acrylate" means "acrylate, methacrylate or a combination of acrylate and methacrylate." The ring-opening polymerization is catalyzed using a lipase enzyme.

Polyester polyol and acrylated polyester polyol -containing compositions that are essentially free of acidic and heavy metal catalyst residues, and which can contain a lipase and/or lipase residues, are also disclosed.

Urethane acrylates prepared using these polyester polyols and/or acrylated polyester polyols and methods of preparing the urethane acrylates are also disclosed. The urethane acrylates are prepared by reacting one or more hydroxy moieties on the polyester polyols with one or more isocyanate moieties on a di- and/or polyisocyanate to form urethane linkages. In one embodiment, the polyester polyol includes at least one (meth)acrylate moiety. In another embodiment, at least one isocyanate moiety in the polyisocyanate is reacted with a hydroxy moiety on a compound that includes at least one hydroxy moiety and at least one (meth)acrylate moiety. In either embodiment, the resulting urethane includes at least one (meth)acrylate moiety. When the polyester polyol is prepared using a lipase catalyst rather than an acidic or heavy metal catalyst, the urethane acrylate is essentially free of acidic or heavy metal catalyst residues.

Coating compositions including the polyester polyols and acrylated polyester polyols and/or urethanes and urethane acrylates prepared using these polyester polyols or acrylated polyester polyols are also disclosed. The coating compositions can be 100% solids coating compositions, water-based coating compositions or solvent-based coating compositions. The compositions can include flatting agents, reactive diluents, photoinitiators, and/or other components suitable for use in coating compositions. In one embodiment, the coatings prepared using the coating compositions are essentially free of acidic and/or heavy metal catalyst residues. The coating compositions can be used to coat surface coverings, such as floor, wall, and ceiling coverings, and such coated floor, wall and ceiling coverings are also disclosed. The "catalyst free" coating compositions can also be water-based, solvent-based and 100% solids UV-curable coating compositions, and can be used to prepare high-performance coatings. The coatings prepared from these coating compositions have improved stability relative to coatings prepared using polyol polymers, acrylated polyols polymers, urethanes or urethane acrylates prepared using heavy metal and/or acid catalysts and that include residues of these catalysts.

In addition to coatings, the polyester polyols and acrylated polyester polyols and/or urethanes and urethane acrylates prepared using these polyester polyols or acrylated polyester polyols, can be used as one or more components in the preparation of various articles of manufacture, such as food wraps or children's toys. Additionally, these polyester polyols can be used to prepare a variety of urethane products that find application in foams, biomedical materials, and thermoplastic polyurethanes.

The polyester polyols and acrylated polyester polyols, methods for preparing these compounds, urethanes and urethane acrylates prepared using these compounds, coating compositions including these compounds and/or urethanes and urethane acrylates prepared using these compounds, and surface coverings and other articles of manufacture prepared from these compounds and compositions, are discussed in more detail below.

The polyester polyol compositions described herein are essentially free of residual acid and/or heavy metals (i.e., the chemistry is "green chemistry" and is environmental-friendly), relative to those prepared using heavy metal and/or acid catalysts. Further, coatings that include these compounds have relatively improved long-term stability, including hydrolysis stability, color stability and light and heat stability when compared to polyol polymers prepared using a catalyst selected from the group consisting of a strong acid catalyst and a metallic catalyst.

Using enzymatic catalysts, the method can be performed at lower temperatures than when heavy metals and/or strong acid catalysts are used. For example, the processing temperature using the enzymatic catalysts is in the range of 70-90°C, whereas a temperature range of 120-160°C is used in connection with acid and/or heavy metal catalysts, with similar reaction times. This results in significant energy savings, and can also result in less color in the final products.

The resulting method provides saturated and unsaturated macro monomers. The saturated and unsaturated macro monomers have a wide application in the coating market. Also, polylactone polyols are one of the major components in the polyurethane market (including thermoplastic polyurethane). For example, polylactone polyols can be reacted with isocyanates and hydroxy-acrylates to form the urethane acrylates in several commercial coating formulations, including Armstrong World Industries' radiation curable Duracote™ coating formulations. In particular, the polyester polyols can be used with a hydroxyacrylate such as Union Carbide's Tone M100 and an isocyanate such as BASF's Desmodur® 3300.

Finally, the ability to avoid metal or acid catalyst residues in UV-curable compositions provides certain additional advantages, such as relatively improved heat and light stability when compared to similar compositions including such residues. Although catalyst residue is not known to pose significant health or safety risks with finished floor coverings or polyurethane coatings, the avoidance of such residues is advantageous when the materials are used in food packaging, children's toys, and medical and biomedical implants. The compositions described herein are essentially free of acidic or heavy metal catalyst residues (i.e., include less than about 50 ppm of these residues).

### I. Production of Polyester Polyols

In one embodiment, the polylactone polyester polyol monomers are prepared via the ring-opening polymerization of lactones, lactide and/or glycolide. This ring-opening reaction is initiated by a hydroxy moiety-containing polymerization initiator (typically a monofunctional alcohol) and catalyzed via a lipase enzyme.

The resulting polylactone polyester polyols typically have a weight average molecular weight in the range of about 146 to about 7000, although molecular weights outside these ranges are within the scope of this invention. The ratio of initiator and the lactone monomer is typically from 1 to 0.067 (1/1 to 1/15) when the hydroxy moiety-containing polymerization initiator is a monofunctional alcohol.

The polyester polyols can include one or more (meth)acrylate moieties, which can be provided by either end-capping the polyester polyols with (meth)acrylate moieties, ideally using a lipase enzyme as the catalyst, or by providing a (meth)acrylate moiety in the hydroxy moiety-containing compounds used in the ring-opening polymerization.

The individual reaction components are described in detail below.

### A. Lactones and Hydroxy-Acids Such as Lactide and Glycolide

Any lactone that is susceptible to enzyme-catalyzed ring-opening polymerization can be used in the method described herein. Examples include epsilon-caprolactone, valerolactone and other 4-alkyl butanolides. Additional representative lactones include those described in U.S. Patent No. 3,655,631.

Cyclic esters of hydroxy acids can also be used in addition to, or in place of, the lactones. Representative examples include lactide and glycolide, as well as lactic acid and glycolic acid.

### B. Initiators

The ring-opening polymerization reaction is essentially a trans-esterification reaction, where the lactone (or lactide or glycolide) is a cyclic ester. To function as an initiator, a compound has to have one hydroxy moiety capable of opening the first lactone moiety, which in turn provides a free hydroxyl moiety that can react with the next lactone moiety, until the polymerization reaction is complete.

An acrylate moiety can be incorporated into the polyester polyol at this stage by using a hydroxy moiety-containing polymerization initiator that includes an acrylate moiety. For example, monofunctional alcohols such as hydroxyalkyl (meth)acrylate, or (R¹)ₐR(OH)_{b} can be used as initiators for the ring-opening polymerization of the lactones to prepare saturated/unsaturated polylactone polyester polyols. In addition to acrylate moieties, other double bond-containing moieties can be used, for example, allyl, alkynyl, vinyl, vinylidene, and vinyl ether.

In the formula (R¹)ₐR(OH)_{b}, a + b > 2, b is at least 1, R¹ is a double bond-containing moiety such as allyl, vinyl, vinylidene, vinyl ether, and acrylate, and R is an alkyl, aryl, aralkyl, alkaryl, ether or ester moiety, including substituted versions thereof. The substituents can be any functional moiety that does not negatively effect the desired enzyme-catalyzed reaction. Examples of suitable substituents include halo, thio, nitrile, nitro, ester, ether, amide, ketone, acetal, silyl, and phosphorous-containing moieties.

R also can be a polymer, including polymers with a plurality of functional moieties. Examples of hydroxyl moiety-containing acrylate compounds include, but are not limited to, 2-hydroxyethyl acrylate, 2-hydroxypropyl acrylate, pentaerythritol triacrylate, and dipentaerythritol pentacrylate.

As used herein, alkyl refers to a straight chain, branched or cyclic alkyl. Heterocyclic moieties can be present provided they do not adversely affect the enzymatic catalysis.

As used herein, alkenyl and alkynyl refer to straight chain, branched or cyclic alkenes and alkynes.

As used herein, aryl refers to a C₆₋₁₀ monocyclic or polycyclic aromatic moiety, including, but not limited to, phenyl, biphenyl, and napthalenyl. Heteroaryl and heterocyclic moieties can be present provided they do not adversely affect the enzymatic catalysis

As used herein, aralkyl refers to an aryl moiety that includes one or more alkyl moieties, where the linkage is directly on the aryl moiety, and alkaryl refers to an alkyl moiety that includes one or more aryl moieties, where the linkage is directly on the alkyl moiety.

### C. Enzymatic Catalysts

Examples of suitable enzymatic lipase catalysts (lipases) include, but are not limited to, porcine pancreatic lipase (PPL), *Candida cylindracea* lipase (CCL), *Pseudomonas fluorescence* lipase (PFL), *Rhizopus javanicuc* lipase (RJL), *Rhizopus delemar* lipase (RDL) and Novozyme 435™.

### D. Solvent Systems

The reaction can be performed in any suitable solvent system. The solvent ideally does not include a significant amount of water beyond that required for the enzyme to function, since the water can compete with the polyol initiators to initiate polymerization of the lactones. The solvent ideally does not include a significant amount of alcohols other than the polyol initiators for the same reason. In those embodiments where the final product is a liquid at the reaction temperature, the reaction can be run neat (i.e., without added solvent).

Ionic liquids and/or supercritical fluid carbon dioxide have both been used as solvents for enzymatic reactions, and can be used in the methods described herein. Organic solvents, such as hexane, heptane, toluene, and xylene can also be used.

### E. Reaction Conditions

As discussed above, the method involves producing a saturated/unsaturated polylactone polyol macromonomer by reacting a lactone and an initiator such as a hydroxyalkyl (meth)acrylate and/or a compound of the Formula (R¹)ₐR(OH)_{b} in the presence of a lipase enzyme catalyst. The ring-opening polymerization reaction generally occurs at standard pressure, and at a temperature less than about 120°C, although higher temperatures can be used. The reaction is typically complete in less than about 24 hours. However, the temperature and time for reaction can be dependent upon the particular catalyst used.

### II. Acrylation of Polyester Polyols.

In the embodiment described above, the polyester polyols are produced with initiators that can but need not include acrylate or other UV-curable moieties. A second way to incorporate such moieties using the lipase catalysts is to first obtain a polyester polyol, and then acrylate one hydroxyl moieties on the polyester polyol using (meth)acrylic acid using the lipase catalyst. Advantageously, the polyester polyol that is used is also prepared using a lipase, so that it does not include any acidic or heavy metal catalyst residues.

### III. Urethanes and Urethane Acrylates Prepared Using Saturated and Unsaturated Polyester Polyols

The saturated and unsaturated polyester polyols described herein can be used to prepare urethanes and urethane acrylates. Where the polyester polyols are saturated, the reaction of a di- or polyisocyanate with the hydroxyl moiety in the polyester polyol forms a urethane. In this reaction, a chain extender, i.e. an amine, may be used. Where the polyester polyols include one or more (meth)acrylate moieties, the reaction of a isocyanate in a di- or polyisocyanate with a hydroxy moiety in the polyester polyols forms a urethane linkage, and the urethanes already include a (meth)acrylate functionality. Where the polyester polyols do not include one or more (meth)acrylate moieties, one or more of the isocyanate moieties can be reacted with a compound that includes a hydroxy moiety and a (meth)acrylate moiety, such as a hydroxy acrylate, so that the urethane molecule includes one or more (meth)acrylate moieties.

The isocyanate compounds used for preparing the urethane acrylates can be selected from the compounds having a linear saturated hydrocarbon, cyclic saturated hydrocarbon, or aromatic hydrocarbon structure. Such isocyanate compounds can be used either individually or in combinations of two or more. The number of isocyanate moieties in a molecule is usually from 1 to 6, and preferably from 1 to 3.

Polyurethanes produced from aromatic isocyanates tend to turn yellowish over time, albeit without a significant loss in mechanical properties. For this reason, in coating applications, it may be desirable to use aliphatic isocyanates. The most widely used aliphatic diisocyanates are 1,6-hexane diisocyanate (HDI), isophorone diisocyanate (IPDI), dicyclohexane diisocyanate (HMDI) also known as hydrogenated MDI, and meta-tetramethylxylene diisocyanate (TMXDI). Additional nonaromatic isocyanates include 1,6,11- undecane triisocyanate, lysine-ethylester triisocyanate, transcyclohexane diisocyanate, and tetramethylxylylene diisocyanate. Toluene diisocyanate and xylylene diisocyanate are examples of aromatic isocyanates.

Representative examples of commercially available polyisocyanate compounds include, but are not limited to, A-1310 and Y-5187 manufactured by Nippon Unicar Co., Ltd.; Calenz MOI manufactured by Showa Denko Co., Ltd.; TDI-80/20, TDI-100, MDI-CR100, MDI-CR300, MDI-PH, and NDI manufactured by Mitsui-Xisso Urethane Co., Ltd.; Coronate T, Millionate MT, Millionate MR, and HDI manufactured by Nippon Polyurethane Industry Co., Ltd.; and Takenate 600 manufactured by Takeda Chemical Industries Co., Ltd.

### IV. Coating Compositions Including the Polyester Polyols and/or Urethane Acrylates Prepared from the Polyester Polyols

The polyester polyols, acrylated polyester polyols and/or urethanes and urethane acrylates prepared from the polyester polyols and acrylated polyester polyols described herein can be used in coating compositions. The coating compositions can also include reactive diluents, photoinitiators, flatting agents, and other components commonly used in coating compositions. The coating compositions can be water-based, organic solvent-based, or 100% solids coating compositions.

### A. Reactive Diluents

The polyester polyols, particularly acrylate polyester polyols, and urethanes and urethane acrylates prepared from the polyester polyols and acrylated polyester polyols, can be combined with suitable reactive diluents to form UV-curable 100 percent solids coating compositions. The reactive diluent(s) are typically low molecular weight (i.e., less than 1000 g/mol), liquid (meth)acrylate-functional compounds. Examples include, but are not limited to: tridecyl acrylate, 1,6-hexanediol diacrylate, 1,4-butanediol diacrylate, ethylene glycol diacrylate, diethylene glycol diacrylate, tetraethylene glycol diacrylate, tripropylene glycol diacrylate and ethoxylated derivatives thereof, neopentyl glycol diacrylate, 1,4-butanediol dimethacrylate, poly(butanediol) diacrylate, tetrathylene glycol dimethacrylate, 1,3-butylene glycol diacrylate, tetraethylene glycol diacrylate, triisopropylene glycol diacrylate, triisopropylene glycol diacrylate, and ethoxylated bisphenol-A diacrylate. Another example of a reactive diluent is N-vinyl caprolactam (International Specialty Products). Further examples are the commercially available products from Sartomer, SR 489, a tridecyl acrylate and SR 506, an isobornyl acrylate.

### B. Flatting Agents

Flatting agents are well known to those of skill in the art, and are used to minimize the gloss levels of coatings. Representative flatting agents include, but are not limited to, silica, nylon, and alumina flatting agents.

### C. Photoinitiators

The compositions can also include a sufficient amount of a free-radical photoinitiator such that the compositions can be UV-cured. Typically, the concentration of photoinitiator is between 1 and 10% by weight, although weight ranges outside of this range can be used. Alternatively, the compositions can be cured using electron beam (EB) curing.
Any compounds that decompose upon exposure to radioactive rays and initiate the polymerization can be used as the photoinitiator in UV-curable compositions including the polyester polyols, acrylated polyester polyols and/or urethane acrylates prepared from the polyester polyols or acrylated polyester polyols. Photosensitizers can be added as desired. The words "radiation" as used in the present invention include infrared rays, visible rays, ultraviolet rays, deep ultraviolet rays, X-rays, electron beams, alpha-rays, beta-rays, and gamma-rays. Representative examples of the photoinitiators include, but are not limited to, acetophenone, acetophenone benzyl ketal, anthraquinone, 1-hydroxycyclohexylphenyl ketone, 2,2-dimethoxy-2-phenylacetophenone, xanthone compounds, triphenylamine, carbazole, 3-methylacetophenone, 4-chlorobenzophenone, 4,4'-dimethoxybenzophenone, 4,4'-diaminobenzophenone, 2-hydroxy-2-methyl-1-phenylpropan-1-one, 1-(4-isopropylphenyl)-2-hydroxy-2-methylpropan-1-one, xanthone, 1,1-dimethoxydeoxybenzoin, 3,3'-dimethyl-4-methoxybenzophenone, thioxanethone compounds, diethylthioxanthone, 2-isopropylthioxanthone, 2-chlorothioxanthone, 1-(4-dodecylphenyl)-2-hydroxy-2-methylpropan-1-one, 2-methyl-1-[4-(methylthio)phenyl]-2-morpholino-propan-1-one, triphenylamine, 2,4,6-trimethylbenzoyldiphenylphosphineoxide, bis-(2,6-dimethoxybenzoyl)-2,4,4-trimethylpentylphosphine oxide, bisacylphosphineoxide, benzyl dimethyl ketal, fluorenone, fluorene, benzaldehyde, benzoin ethyl ether, benzoin propyl ether, benzophenone, Michler's ketone, 2-benzyl-2-dimethylamino-1- (4-morpholinophenyl)-butan-1-one, 3-methylacetophenone, and 3,3',4,4'-tetra(t-butylperoxycarbonyl)benzophenone (BTTB). Commercially available photoinitiators include, but are not limited to, Irgacure® 184, 651, 500, 907, 369, 784 and 2959 (manufactured by Ciba Specialty Chemicals Co., Ltd.), Lucirine TPO (manufactured by BASF), Darocur® 1116 and 1173 (manufactured by Ciba Specialty Chemicals Co., Ltd.), Lucirine TPO (manufactured by BASF), Ubecryl® P36 (manufactured by UCB), and Escacure® KIP150, KIP100F (manufactured by Lamberti).
Representative examples of photosensitizers include, but are not limited to, triethylamine, diethylamine, N-methyldiethanolamine, ethanolamine, 4-dimethylaminobenzoic acid, methyl 4-dimethylaminobenzoate, ethyl 4-dimethylaminobenzoate, and isoamyl 4-dimethylaminobenzoate, as well as commercially available products such as Ubecryl® P102, 103, 104,and 105 (manufactured by UCB).
The photoinitiators are typically present in the range of from 0.01 to 10 wt %, although amounts outside this range can be used. Thermal initiators, such as AIBN and di-t-butyl peroxide can be used in place of or in addition to the photoinitiators.
The coating compositions can be used to coat surface coverings, such as floor, wall and ceiling coverings. The coating compositions can be applied to surface coverings using any application method suitable for use with urethane acrylate coating compositions. Such methods include roll coating, spray coating, and dip coating. Such coatings are essentially free of acidic and/or heavy metal catalyst residues, and as such, are more stable than coatings including such residues.
The resulting coated products can be tested for improved stability, including stability to both heat and light. Standard ASTM method F1514-98 can be used, advantageously with minor modifications. The standard ASTM method calls for evaluating the material at 158°F for 7 days and comparing color differences. Advantageously, this test is extended to 6 weeks, with results being determined at every week interval.
Light stability testing can be evaluated using ASTM F1515, advantageously with minor modifications. The standard ASTM method involves exposing the material to a Xenon light source and reporting any differences in color readings at 100, 200, 300 and 400 hours. Advantageously, this test is extended to 500 and 600 hrs.
Improvements in stability can be evaluated by making identical compositions wherein the only difference in the composition is the catalyst used to make the polyester polyol or acrylated polyester polyol materials. That is, one sample can be prepared via enzymatic catalysis, and the second sample prepared via conventional acid or metal-based catalysis.

### V. Articles of Manufacture Prepared Using the Polyester Polyols and Acrylated Polyester Polyols and/or Urethanes and Urethane Acrylates Prepared from the Polyester Polyols

The polyester polyols, acrylated polyester polyols, urethanes, and/or urethane acrylates can be used to prepare any article of manufacture commonly made using these components. They can be present in plastics used in food packaging, such as food containers and films used to wrap food products, children's toys, and medical and biomedical implants. In one embodiment, the compounds and/or compositions including the compounds are added to a mold and polymerized to form a desired article of manufacture.

The present invention will be better understood with reference to the following nonlimiting examples.

### Comparative Example 1: Preparation of a hydroxyl-terminated polyester triol:

One hundred forty-eight grams of epsilon-caprolactone, 26 grams of trimethylolpropane and 3 grams porcine pancrease lipase (type II) were charged into 250 ml glass reaction vessel fitted with a mechanical stirrer, condenser, thermometer and dry air inlet and outlet tubes. The mixture was stirred and heated to 70°C with blanketed dry air at 0.10 SCFH (0.283 cubic meter/hour at 273.15 °K (0°C) and one atmosphere of pressure (760 torr or 101.325 kilopascals)). The reaction mixture was stirred at 70°C. Gas chromatography analysis was used to monitor epsilon-caprolactone conversion. After 4 hours of reaction time, the epsilon-caprolactone conversion was 98.5% complete. After 6 hours, the reaction was 98.7% complete. The reaction mixture was then cooled down to room temperature. The final product was collected by filtering off the enzyme catalyst.

### Example 2: Preparation of Unsaturated polyester polyols

Six hundred twenty-six grams of epsilon-caprolactone, 318 grams of 2-hydroxyethyl acrylate, 0.68 grams monomethyl ether hydroquinone (p-methoxyphenol) and 9 grams Novozyme-435 were charged into 1L glass reaction vessel fitted with a mechanical stirrer, condenser, thermometer and dry air inlet and outlet tubes. The mixture was stirred and heated to 70°C with blanketed dry air (0.10 SCFH (0.283 cubic meter/hour at 273.15 °K (0°C) and one atmosphere of pressure (760 torr or 101.325 kilopascals))). The reaction mixture was stirred at 70°C. Gas chromatography analysis was used to monitor epsilon-caprolactone conversion. After 4 hours, the epsilon-caprolactone conversion was 99.6% complete. After 6 hours, there was no change in the amount of conversion. The reaction mixture was then cooled down to room temperature. The final product was collected by filtering off the enzyme catalyst.

### Example 3: Preparation of Multi-functional unsaturated polyester polyols

One hundred twenty four grams of epsilon-caprolactone, 62 grams trimethylolpropane diallyl ether, 0.14 grams monomethyl ether hydroquinone (p-methoxyphenol) and 2 grams Novozyme-435 were charged into 250 ml glass reaction vessel fitted with a mechanical stirrer, condenser, thermometer and dry air inlet and outlet tubes. The mixture was stirred and heated to 70°C with blanketed dry air (0.10 SCFH (0.283 cubic meter/hour at 273.15 °K (0°C) and one atmosphere of pressure (760 torr or 101.325 kilopascals))). The reaction mixture was stirred at 70°C. Gas chromatography analysis was used to monitor epsilon-caprolactone conversion. After 4 hours, the epsilon-caprolactone conversion was 98% complete. There was no change after 6 hours. The reaction mixture was then cooled down to room temperature. The final product was collected by filtering off the enzyme catalyst.

### Example 4: Preparation of acrylated polyester polyol

Two hundred sixty-seven grams of a hydroxy-terminated polyester (a polymer prepared from composition containing 59.3% by weight of 1,6 hexanediol, 15.6% phthalic anhydride, and 25.1% trimellitic anhydride, where the acid number is less than 5), 92 grams of acrylic acid, 0.04 grams monomethyl ether hydroquinone (p-methoxyphenol), 0.04 grams hydroquinone, 36 grams Novozyme-435 and 65 ml heptane were charged into 1000 ml glass reaction vessel fitted with a mechanical stirrer, D-S trap and water condenser connected to a vacuum system, thermometer and dry air inlet. The mixture was stirred and heated to 90°C with blanketed dry air at 0.10 SCFH (0.283 cubic meter/hour at 273.15 °K (0°C) and one atmosphere of pressure (760 torr or 101.325 kilopascals)). Then, the dry airflow was stopped and vacuum was gradually applied to initiate boiling. The boiling was adjusted to maximum and the aqueous layer formed in phase separation was removed. Additional heptane was added as needed due to heptane loss. After 6 hours, the final product was recovered by vacuum stripping off the solvent and excess acrylic acid, and filtering off the enzyme catalyst. The product, acrylated polyester polyol, had a hydroxy number of 88.

### Example 5: Coating formulation

Polycaprolactone triols, unsaturated polyol and acrylated polyester polyol obtained from comparative Example 1,and Examples 2 & 4 were formulated into a dual (thermal/UV) cure coating. See formulation in Table 1. The coating was coated on a vinyl floor, thermally cured at 190°C for 2 minutes, then UV-cured at 2J/cm². The final coating had very good stain resistance and abrasion resistance without containing any metal residue.

**Table 1**

| | |
|---|---|
| Acrylated polyester polyol (Example 4) | 70.00 gr |
| Unsaturated polyol (Example 2) | 15.00 gr |
| Polyester polyol (comparative Example 1) | 15.00 gr |
| 2-hydroxyethyl acrylate | 15.00 gr |
| Resimene® CE-7103 | 51.87 gr |
| DC-57 | 0.50 gr |
| Benzophenone | 3.01 gr |
| KIP-100F | 2.01 gr |
| Nacure® XP-357 | 6.69 gr |

or 101.325 kilopascals)). The reaction mixture was stirred at 70°C. Gas chromatography analysis was used to monitor epsilon-caprolactone conversion. After 4 hours of reaction time, the epsilon-caprolactone conversion was 98.5% complete. After 6 hours, the reaction was 98.7% complete. The reaction mixture was then cooled down to room temperature. The final product was collected by filtering off the enzyme catalyst.

### Example 2: Preparation of Unsaturated polyols

Six hundred twenty-six grams of epsilon-caprolactone, 318 grams of 2-hydroxyethyl acrylate, 0.68 grams monomethyl ether hydroquinone (p-methoxyphenol) and 9 grams Novozyme-435 were charged into 1L glass reaction vessel fitted with a mechanical stirrer, condenser, thermometer and dry air inlet and outlet tubes. The mixture was stirred and heated to 70°C with blanketed dry air (0.10 SCFH (0.283 cubic meter/hour at 273.15 °K (0°C) and one atmosphere of pressure (760 torr or 101.325 kilopascals))). The reaction mixture was stirred at 70°C. Gas chromatography analysis was used to monitor cpsilon-caprolactone conversion. After 4 hours, the epsilon-caprolactone conversion was 99.6% complete. After 6 hours, there was no change in the amount of conversion. The reaction mixture was then cooled down to room temperature. The final product was collected by filtering off the enzyme catalyst.

### Example 3: Preparation of Multi-functional unsaturated polyols

One hundred twenty four grams of epsilon-caprolactone, 62 grams trimethylolpropane diallyl ether, 0.14 grams monomethyl ether hydroquinone (p-methoxyphenol) and 2 grams Novozyme-435 were charged into 250 ml glass reaction vessel fitted with a mechanical stirrer, condenser, thermometer and dry air inlet and outlet tubes. The mixture was stirred and heated to 70°C with blanketed dry air (0.10 SCFH (0.283 cubic meter/hour at 273.15 °K (0°C) and one atmosphere of pressure (760 torr or 101.325 kilopascals))). The reaction mixture was stirred at 70°C. Gas chromatography analysis was used to monitor epsilon-caprolactone conversion. After 4 hours, the epsilon-caprolactone conversion was 98% complete. There was no change after 6 hours. The reaction mixture was then cooled down to room temperature. The final product was collected by filtering off the enzyme catalyst.

### Example 4: Preparation of acrylated polyester polyol

Two hundred sixty-seven grams of a hydroxy-terminated polyester (a polymer prepared from composition containing 59.3% by weight of 1,6 hexanediol, 15.6% phthalic anhydride, and 25.1% trimellitic anhydride, where the acid number is less than 5), 92 grams of acrylic acid, 0.04 grams monomethyl ether hydroquinone (p-methoxyphenol), 0.04 grams hydroquinone, 36 grams Novozyme-435 and 65 ml heptane were charged into 1000 ml glass reaction vessel fitted with a mechanical stirrer, D-S trap and water condenser connected to a vacuum system, thermometer and dry air inlet. The mixture was stirred and heated to 90°C with blanketed dry air at 0.10 SCFH (0.283 cubic meter/hour at 273.15 °K (0°C) and one atmosphere of pressure (760 torr or 101.325 kilopascals)). Then, the dry airflow was stopped and vacuum was gradually applied to initiate boiling. The boiling was adjusted to maximum and the aqueous layer formed in phase separation was removed. Additional heptane was added as needed due to heptane loss. After 6 hours, the final product was recovered by vacuum stripping off the solvent and excess acrylic acid, and filtering off the enzyme catalyst. The product, acrylated polyester polyol, had a hydroxy number of 88.

### Example 5: Coating formulation

Polycaprolactone triols, unsaturated polyol and acrylated polyester polyol obtained from Examples 1, 2 & 4 were formulated into a dual (thermal/UV) cure coating. See formulation in Table 1. The coating was coated on a vinyl floor, thermally cured at 190°C for 2 minutes, then UV-cured at 2J/cm². The final coating had very good stain resistance and abrasion resistance without containing any metal residue.

**Table 1**

| | |
|---|---|
| Acrylated polyester polyol (Example 4) | 70.00 gr |
| Unsaturated polyol (Example 2) | 15.00 gr |
| Polyester polyol (Example 1) | 15.00 gr |
| 2-hydroxyethyl acrylate | 15.00 gr |
| Resimene® CE-7103 | 51.87 gr |
| DC-57 | 0.50 gr |
| Benzophenone | 3.01 gr |
| KIP-100F | 2.01 gr |
| Nacure® XP-357 | 6.69 gr |

## Claims

1. A polyester polyol composition comprising:
a) a polyester polyol comprising a plurality of moieties derived from a compound selected from the group consisting of a lactone, a lactide, a glycolide, and combinations thereof, wherein at least one of the plurality of moieties is covalently linked via an ester linkage to the hydroxy moiety of a hydroxy moiety-containing polymerization initiator, the hydroxy moiety-containing polymerization initiator comprising ethylenic unsaturation, and
b) an enzyme catalyst.

2. The polyester polyol composition according to claim 1, wherein the composition is essentially free of acidic catalyst residues and metallic catalyst residues.

3. The polyester polyol composition according to claim 1 or 2, wherein the enzyme catalyst comprises a lipase enzyme.

4. The polyester polyol composition according to any of the preceding claims, further comprising a hydroxy moiety-containing polymerization initiator of the formula (R¹)ₐR(OH)_{b},
wherein a + b ≥2, a is at least 1 and b is at least 1,
R¹ is a double bond-containing moiety, and
R is selected from the group consisting of optionally substituted alkyl, aryl, aralkyl, alkaryl, ether and ester moieties, and a polymer comprising at least one hydroxy moiety, wherein the optional substituent is selected from the group consisting of halo, thio, nitrile, nitro, ester, ether, amide, ketone, acetal, silyl, phosphorous, and combinations thereof.

5. The polyester polyol composition according to claim 1, wherein the ethylenic unsaturation is present in a moiety selected from the group consisting of a (meth)acrylate moiety, an allyl moiety, a vinyl moiety, a vinylidine moiety, a vinyl ether moiety, and an alkynyl moiety.

6. The polyester polyol composition according to claim 5, wherein the polymerization initiator comprises a (meth)acrylate moiety.

7. The polyester polyol composition according to claim 1, wherein the hydroxy moiety-containing polymerization initiator is selected from the group consisting of 2-hydroxyethyl acrylate, 2-hydroxypropyl acrylate, pentaerythritol triacrylate, dipentaerythritol pentacrylate, and combinations thereof.

8. The polyester polyol composition according to any of the preceding claims, wherein the lactone is selected from the group consisting of caprolactone and valerolactone.

9. The polyester polyol composition according to any of the preceding claims, wherein the polyester polyol further comprises at least one ester moiety derived from a hydroxy moiety on the polyester polyol and (meth)acrylic acid.

10. A composition comprising a compound selected from the group consisting of a urethane monomer, a urethane polymer, and combinations thereof, wherein the urethane moiety comprises urethane linkages formed from at least one hydroxy moiety on a polyester polyol according to any of the claims 1 to 9, the polyester polyol being prepared via enzyme-catalyzed ring-opening transesterification being initiated by a hydroxy moiety-containing polymerization initiator comprising ethylenic unsaturation, with at least one isocyanate moiety on a polyisocyanate.

11. The composition according to claim 10, wherein the composition further comprises a reactive diluent.

12. The composition according to any of claims 10 or 11, wherein the composition further comprises a flatting agent.

13. The composition according to any of the claims 10 to 12, wherein the composition further comprises a photoinitiator.

14. The composition according to any of the claims 1 to 13 in the form of a coating comprising a (meth)acrylate polyester polyol, wherein the polyester polyol is prepared by a method comprising a step selected from the group consisting of:
(a) forming a polyester polyol by ring-opening polymerization of a compound selected from the group consisting of a lactone, a lactide, a glycolide, and combinations thereof, the ring opening being initiated by a hydroxy moiety-containing polymerization initiator comprising a (meth)acrylate moiety, wherein the ring-opening polymerization reaction is catalyzed by an enzymatic catalyst,
(b) capping at least one of the end of a polyester polyol with a (meth)acrylate moiety, wherein the end capping reaction is catalyzed by an enzymatic catalyst, and
(c) the combination of steps (a) and (b).

15. A surface covering coated with the composition according to any of the claims 1 to 14.

16. The surface covering according to claim 15, wherein the surface covering is a floor covering.

17. A method for preparing the composition according to any of claims 1 to 9, comprising forming a polyester polyol by
the step of ring-opening polymerization of a compound selected from the group consisting of a lactone, a lactide, a glycolide, and combinations thereof, the ring opening being initiated by a hydroxy moiety-containing polymerization initiator comprising ethylenic unsaturation, wherein the ring-opening polymerization reaction is catalyzed by an enzymatic catalyst.

18. The method according to claim 17, wherein the enzyme catalyst comprises a lipase.

19. The method according to claims 17 or 18, wherein the polyester polyol comprises at least two ends, and wherein the method further comprises the step of capping at least one of the ends with a (meth)acrylate moiety.

20. The method according to claim 19, wherein the capping step is catalyzed by an enzyme catalyst.

21. The method according to claim 20, wherein the enzyme catalyst for the capping step comprises a lipase.

22. The method according to any of claims 17 to 21, wherein the compound including at least two hydroxy moieties further comprises a double bond-containing moiety.

23. A method for forming a urethane acrylate, comprising reacting a polyester polyol according to claims 1 to 9 with a polyisocyanate comprising:
a) preparing a polyester polyol comprising at least one (meth)acrylate moiety by a ring-opening polymerization of a compound selected from the group consisting of a lactone, a lactide, a glycolide, and combinations thereof, the ring opening being initiated by a hydroxy moiety-containing polymerization initiator comprising ethylenic unsaturation, wherein the ring opening polymerization is catalyzed by an enzymatic catalyst; and
b) reacting at least one isocyanate moiety on a polyisocyanate with a hydroxy moiety on the polyester polyol.

## Patentansprüche

1. Polyesterpolyolzusammensetzung, enthaltend:
a) ein Polyesterpolyol, das eine Vielzahl von Fragmenten, erhalten aus einer Verbindung, ausgewählt aus der Gruppe, bestehend aus einem Lacton, einem Lactid, einem Glycolid und Gemischen davon, umfaßt, wobei wenigstens ein Fragment aus der Vielzahl von Fragmenten über eine Esterbindung mit dem Hydroxyfragment eines ein Hydroxyfragment enthaltenden Polymerisationsinitiators kovalent verknüpft ist, wobei der ein Hydroxyfragment enthaltende Polymerisationsinitiator eine ethylenische Ungesättigtheit umfaßt, und
b) einen Enzymkatalysator.

2. Polyesterpolyolzusammensetzung nach Anspruch 1, wobei die Zusammensetzung von sauren Katalysatorresten und metallischen Katalysatorresten im Wesentlichen frei ist.

3. Polyesterpolyolzusammensetzung nach Anspruch 1 oder 2, wobei der Enzymkatalysator ein Lipaseenzym umfaßt.

4. Polyesterpolyolzusammensetzung nach einem der vorhergehenden Ansprüche, die außerdem noch einen ein Hydroxyfragment enthaltenden Polymerisationsinitiator der Formel (R¹)ₐR(OH)_{b}, worin
a + b≥ 2, a mindestens 1 und b mindestens 1 und
R¹ ein eine Doppelverbindung enthaltendes Fragment bedeuten, und
R ausgewählt ist aus der Gruppe, bestehend aus gegebenenfalls substituierten Alkyl-, Aryl-, Aralkyl-, Alkaryl-, Ether- und Esterfragmenten und ein Polymer umfaßt, das wenigstens ein Hydroxyfragment umfaßt, wobei der gegebenenfalls verwendete Substituent ausgewählt wird aus der Gruppe, bestehend aus Halogen, Thio, Nitril, Nitro, Ester, Ether, Amid, Keton, Acetal, Silyl, Phosphor und Gemischen davon.

5. Polyesterpolyolzusammensetzung nach Anspruch 1, wobei die ethylenische Ungesättigtheit vorliegt in einem Fragment, ausgewählt aus der Gruppe, bestehend aus einem (Meth)acrylat-, einem Allkyl-, einem Vinyl-, einem Vinyliden-, einem Vinylether- und einem Alkinylfragment.

6. Polyesterpolyolzusammensetzung nach Anspruch 5, wobei der Polymerisationsinitiator ein (Meth)acrylatfragment umfaßt.

7. Polyesterpolyolzusammensetzung nach Anspruch 1, wobei der ein Hydroxyfragment enthaltende Polymerisationsinitiator ausgewählt ist aus der Gruppe, bestehend aus 2-Hydroxyethylacrylat, 2-Hydroxypropylacrylat, Pentaerythrittriacrylat, Dipentaerythritpentacrylat und Gemischen davon.

8. Polyesterpolyolzusammensetzung nach einem der vorhergehenden Ansprüche, wobei das Lacton ausgewählt ist aus der Gruppe, bestehend aus Caprolacton und Valerolacton.

9. Polyesterpolyolzusammensetzung nach einem der vorhergehenden Ansprüche, wobei das Polyesterpolyol außerdem noch wenigstens ein Esterfragment, erhalten aus einem Hydroxyfragment am Polyesterpolyol und (Meth)acrylsäure, umfaßt.

10. Zusammensetzung, umfassend eine Verbindung, ausgewählt aus der Gruppe, bestehend aus einem Urethanmonomer, einem Urethanpolymer und Gemischen davon, wobei das Urethanfragment Urethanbindungen umfaßt, gebildet aus wenigstens einem Hydroxyfragment an einem Polyesterpolyol nach einem der Ansprüche 1 bis 9, und das Polyesterpolyol hergestellt wird durch enzymkatalysierte Ringöffnungsumesterung, initiiert durch einen ein Hydroxyfragment enthaltenden Polymerisationsinitiator, umfassend eine ethylenische Ungesättigtheit, mit wengistens einem Isocyanatfragment an einem Polyisocyanat.

11. Zusammensetzung nach Anspruch 10, wobei die Zusammensetzung außerdem noch ein reaktionsfähiges Verdünnungsmittel umfaßt.

12. Zusammensetzung nach einem der Ansprüche 10 oder 11, wobei die Zusammensetzung außerdem noch ein Mattierungsmittel umfaßt.

13. Zusammensetzung nach einem der Ansprüche 10 bis 12, wobei die Zusammensetzung außerdem noch einen Photoinitiator umfaßt.

14. Zusammensetzung nach einem der Ansprüche 1 bis 13, in Form einer Beschichtung, die ein (Meth)acrylatpolyesterpolyol umfaßt, wobei das Polyesterpolyol hergestellt wird nach einem Verfahren, das eine Stufe umfaßt, ausgewählt aus der Gruppe, bestehend aus:
(a) Bildung eines Polyesterpolyols durch Ringöffnungspolymerisation einer Verbindung, ausgewählt aus der Gruppe, bestehend aus einem Lacton, einem Lactid, einem Glycolid und Gemischen davon, wobei die Ringöffnung durch einen ein Hydroxyfragment enthaltenden Polymerisationsinitiator, umfassend ein (Meth)acrylatfragment, initiiert wird, und die Ringöffnungspolymerisationsreaktion durch einen enzymatischen Katalysator katalysiert wird,
(b) Absättigung wenigstens eines Endes eines Polyesterpolyols mit einem (Meth)acrylatfragments, wobei die Reaktion der Absättigung des Endes durch einen enzymatischen Katalysator katalysiert wird, und
(c) die Kombination aus den Stufen (a) und (b).

15. Oberflächenbelag, beschichtet mit der Zusammensetzung nach einem der Ansprüche 1 bis 14.

16. Oberflächenbelag nach Anspruch 15, bei dem dieser ein Bodenbelag ist.

17. Verfahren zur Herstellung der Zusammensetzung nach einem der Ansprüche 1 bis 9, das die Bildung eines Polyesterpolyols durch Ringöffnungspolymerisation einer Verbindung, ausgewählt aus der Gruppe, bestehend aus einem Lacton, einem Lactid, einem Glycolid und Gemischen davon, wobei die Ringöffnung durch einen ein Hydroxyfragment enthaltenden Polymerisationsinitiator, umfassend eine ethylenische Ungesättigtheit initiiert wird, und die Ringöffnungspolymerisationsreaktion durch einen enzymatischen Katalysator katalysiert wird, umfaßt.

18. Verfahren nach Anspruch 17, bei dem der Enzymkatalysator eine Lipase umfaßt.

19. Verfahren nach Anspruch 17 oder 18, bei dem das Polyesterpolyol wenigstens zwei Enden umfaßt, und das Verfahren außerdem noch die Stufe der Absättigung wenigstens eines der Enden mit einem (Methy)acrylatfragment umfaßt.

20. Verfahren nach Anspruch 19, bei dem die Absättigungsstufe durch einen Enzymkatalysator katalysiert wird.

21. Verfahren nach Anspruch 20, bei dem der Enzymkatalysator für die Absättigungsstufe eine Lipase umfaßt.

22. Verfahren nach Anspruch 17 bis 21, bei dem die wenigstens zwei Hydroxyfragmente umfassende Verbindung außerdem noch ein eine Doppelbindung enthaltendes Fragment umfaßt.

23. Verfahren zur Herstellung eines Urethanacrylats, das die Umsetzung eines Polyesterpolyols nach den Ansprüchen 1 bis 9 mit einem Polyisocyanat umaßt, umfassend:
a) die Herstellung eines Polyesterpolyols, das wenigstens ein (Meth)acrylatfragment umfaßt, durch eine Ringöffnungspolymerisation einer Verbindung, ausgewählt aus der Gruppe, bestehend aus einem Lacton, einem Lactid, einem Glycolid und Gemischen davon, wobei die Ringöffnung durch einen ein Hydroxyfragment enthaltenden Polymerisationsinitiator, umfassend eine ethylenische Ungesättigtheit und durch ein (Meth)acrylatfragment initiiert wird, und die Ringöffnungspolymerisationsreaktion durch einen enzymatischen Katalysator katalysiert wird, und
b) die Umsetzung wenigstens Isocyanatfragments an einem Polyisocyanat mit einem Hydroxyfragment am Polyesterpolyol.

## Revendications

1. Composition de polyesterpolyol comprenant :
a) un polyesterpolyol comprenant plusieurs fractions qui dérivent d'un composé choisi parmi le groupe constitué par une lactone, un lactide, un glycolide et leurs combinaisons, au moins une desdites plusieurs fractions étant liée de manière covalente via une liaison ester à la fraction hydroxyle d'un initiateur de polymérisation contenant une fraction hydroxyle, initiateur de polymérisation contenant une fraction hydroxyle comprenant une insaturation éthylénique ; et
b) un catalyseur enzymatique.

2. Composition de polyesterpolyol selon la revendication 1, dans laquelle la composition est essentiellement exempte de résidus de catalyseurs acides et de résidus de catalyseurs métalliques.

3. Composition de polyesterpolyol selon la revendication 1 ou 2, dans laquelle le catalyseur enzymatique comprend une lipase à titre d'enzyme.

4. Composition de polyesterpolyol selon l'une quelconque des revendications précédentes, comprenant en outre un initiateur de polymérisation contenant une fraction hydroxyle, répondant à la formule (R¹)ₐR(OH)_{b}, dans laquelle
a + b ≥ 2, a est égal à au moins 1 et b est égal à au moins 1 ;
R¹ représente une fraction contenant une liaison double ; et
R est choisi parmi le groupe constitué par une fraction alkyle, une fraction aryle, une fraction aralkyle, une fraction alkaryle, une fraction éther et une fraction ester, facultativement substituées, et un polymère comprenant au moins une fraction hydroxyle, le substituant facultatif étant choisi parmi le groupe constitué par un atome d'halogène, un groupe thio, un groupe nitrile, un groupe nitro, un groupe ester, un groupe éther, un groupe amide, un groupe cétone, un groupe acétal, un groupe silyle, un groupe phosphore et leurs combinaisons.

5. Composition de polyesterpolyol selon la revendication 1, dans laquelle l'insaturation éthylénique est présente dans une fraction choisie parmi le groupe constitué par une fraction (méth)acrylate, une fraction allyle, une fraction vinyle, une fraction vinylidène, une fraction d'éther vinylique et une fraction alcynyle.

6. Composition de polyesterpolyol selon la revendication 5, dans laquelle l'initiateur de polymérisation comprend une fraction (méth)acrylate.

7. Composition de polyesterpolyol selon la revendication 1, dans laquelle l'initiateur de polymérisation contenant une fraction hydroxyle est choisi parmi le groupe constitué par l'acrylate de 2-hydroxyéthyle, l'acrylate de 2-hydroxypropyle, le triacrylate de pentaérythritol, le pentacrylate de dipentaérythritol, et leurs combinaisons.

8. Composition de polyesterpolyol selon l'une quelconque des revendications précédentes, dans laquelle la lactone est choisie parmi le groupe constitué par la caprolactone et la valérolactone.

9. Composition de polyesterpolyol selon l'une quelconque des revendications précédentes, dans laquelle le polyesterpolyol comprend en outre au moins une fraction ester qui dérive d'une fraction hydroxyle sur le polyesterpolyol et de l'acide (méth)acrylique.

10. Composition comprenant un composé choisi parmi le groupe constitué par un monomère d'uréthane, un polymère d'uréthane et leurs combinaisons, dans laquelle la fraction d'uréthane comprend des liaisons uréthane que l'on obtient à partir d'au moins une fraction hydroxyle sur un polyesterpolyol selon l'une quelconque des revendications 1 à 9, le polyesterpolyol étant préparé via une transestérification par ouverture de cycle catalysée par une enzyme, déclenchée par un initiateur de polymérisation contenant une fraction hydroxyle comprenant une insaturation éthylénique, avec au moins une fraction isocyanate sur un polyisocyanate.

11. Composition selon la revendication 10, dans laquelle la composition comprend en outre un diluant réactif.

12. Composition selon l'une quelconque des revendications 10 ou 11, dans laquelle la composition comprend en outre un agent de matage.

13. Composition selon l'une quelconque des revendications 10 à 12, dans laquelle la composition comprend en outre un photoinitiateur.

14. Composition selon l'une quelconque des revendications 1 à 13 sous la forme d'une enduction comprenant un polyesterpolyol de (méth)acrylate, le polyesterpolyol étant préparé via un procédé comprenant une étape choisie parmi le groupe constitué par :
(a) la formation d'un polyesterpolyol par polymérisation par ouverture de cycle, d'un composé choisi parmi le groupe constitué par une lactone, un lactide, un glycolide et leurs combinaisons, l'ouverture de cycle étant déclenchée par un initiateur de polymérisation contenant une fraction hydroxyle, comprenant une fraction (méth)acrylate, la réaction de polymérisation par ouverture de cycle étant catalysée par un catalyseur enzymatique,
(b) le coiffage d'au moins une des extrémités d'un polyesterpolyol avec une fraction (méth)acrylate, la réaction de coiffage terminal étant catalysée par un catalyseur enzymatique ; et
(c) la combinaison des étapes (a) et (b).

15. Revêtement de surface enduit avec la composition selon l'une quelconque des revendications 1 à 14.

16. Revêtement de surface selon la revendication 15, dans lequel le revêtement de surface est un revêtement de sol.

17. Procédé pour la préparation de la composition selon l'une quelconque des revendications 1 à 9, comprenant la formation d'un polyesterpolyol via l'étape de polymérisation par ouverture de cycle, d'un composé choisi parmi le groupe constitué par une lactone, un lactide, un glycolide et leurs combinaisons, l'ouverture de cycle étant déclenchée par un initiateur de polymérisation contenant une fraction hydroxyle, comprenant une insaturation éthylénique, la réaction de polymérisation par ouverture de cycle étant catalysée par un catalyseur enzymatique.

18. Procédé selon la revendication 17, dans lequel le catalyseur enzymatique comprend une lipase.

19. Procédé selon la revendication 17 ou 18, dans lequel le polyesterpolyol comprend au moins deux extrémités, et dans lequel le procédé comprend en outre l'étape de coiffage d'au moins une des extrémités avec une fraction (méth)acrylate.

20. Procédé selon la revendication 19, dans lequel l'étape de coiffage est catalysée par un catalyseur enzymatique.

21. Procédé selon la revendication 20, dans lequel le catalyseur enzymatique pour l'étape de coiffage comprend une lipase.

22. Procédé selon l'une quelconque des revendications 17 à 21, dans lequel le composé englobant au moins deux fractions hydroxyle comprend en outre une fraction contenant une liaison double.

23. Procédé pour former un acrylate d'uréthane, comprenant la mise en réaction d'un polyesterpolyol selon les revendications 1 à 9 avec un polyisocyanate, comprenant :
a) la préparation d'un polyesterpolyol comprenant au moins une fraction (méth)acrylate via une polymérisation par ouverture de cycle, d'un composé choisi parmi le groupe constitué par une lactone, un lactide, un glycolide et leurs combinaisons, l'ouverture de cycle étant déclenchée par un initiateur de polymérisation contenant une fraction hydroxyle, comprenant une insaturation éthylénique, la polymérisation par ouverture de cycle étant catalysée par un catalyseur enzymatique ; et
b) la mise en réaction d'au moins une fraction isocyanate sur un polyisocyanate avec une fraction hydroxyle sur le polyesterpolyol.
